# EUROPEAN PATENT APPLICATION

(11) **EP 1 361 286 A1**
(43) Date of publication of application: **12.11.2003**
(21) Application number: 02008773.0
(22) Date of filing: 18.04.2002
(51) Int. Cl.: C12Q 1/68

(54) **Method for detection of cancer**

(71) Applicant: MTM Laboratories AG, 69120 Heidelberg (DE)
(72) Inventor: Von Knebel Doeberitz, M., chir. Universitätsklinik, 69120 Heidelberg (DE); Gebert, Johannes, 69221 Heidelberg (DE); Linnebacher, Michael, 66578 Stennweiler (DE); Wörner, Stefan, 69115 Heidelberg (DE); Ridder Rüdiger, 69198 Schriessheim (DE); Bork, Peer, 69117 Heidelberg (DE); Yuan, Y. R., 69118 Heidelberg (DE)
(74) Representative: Schüssler, Andrea, Dr.

(57) **Abstract**

The present invention relates to a method for diagnosis and prognostic assessment of disorders associated with frameshift polypeptides originating from frameshift mutations in coding microsatellite regions of genes based on the detection of immunological entities directed against said frameshift polypeptides in body fluids.

## Description

The present invention relates to a method for diagnosis and prognostic assessment of disorders associated with frameshift polypeptides originating from frameshift mutations in coding microsatellite regions of genes based on the detection of immunological entities directed against said frameshift polypeptides in body fluids.

Tumour cells accumulate mutations in components of cellular pathways, that are essential for the maintenance of normal growth and differentiation. In human epithelial tumors, 2 types of genetic instability have been identified: chromosomal instability (CIN), which marks structural and numerical chromosomal aberration in aneuploid neoplastic cells, and microsatellite instability (MSI), which reflects length variations at repetitive DNA sequences in tumour cells. The type and spectrum of mutated genes markedly differs among CIN and MSI tumours, suggesting distinct but not mutually exclusive pathways of carcinogenesis. MSI occurs in about 90% of hereditary non-polyposis colorectal cancers (HNPCC) as well as in about 15% of sporadic tumours of the colon and other organs, and is caused by mutational inactivation of different DNA mismatch repair genes.

The mutations lead especially in the case of frameshift mutations in coding microsatellite regions to the expression of new peptide sequences in the affected cells, that do not occur in wild-type cells. The altered peptides may be used as detection markers for disorders associated with frameshift mutations in coding microsatellite regions such as degenerative disorders or cancers (e.g. gastrointestinal cancers).

One problem with the detection of the respective marker molecules expressed by mutated cells, is, that they may be not easily accessible. Regarding all disorders located within the body of individuals rather than on an accessible surface there are problems in sample preparation. This is especially crucial for early detection and screening tests. For these purposes the characteristic gene-products originating from a small number of mutated cells have to be detected. When excretions or body fluids are used as samples for detection methods, the potentially mutated cells affected by the disorder in question may be dispersed throughout the whole sample (e.g. gastrointestinal secretions), or may be concentrated in special locations within the sample (e.g. stool). Thus the relevant information about a disorder may easily escape detection.

In the art a variety of approaches to enhance sample preparation has been proposed. These approaches comprise rather sophisticated procedures for the preparation of sample from gastrointestinal secretions or from body fluids, to minimize the probability of the marker molecules to escape detection.

The methods for detection of molecular markers suitable for diagnosis of disorders associated with frameshift mutations in coding microsatellite regions and for assessment of prognosis for said disorders are prone to overlook especially markers originating from small populations of affected cells as they may occur particularly in early stages of the disorder. Furthermore the methods require an elevated effort in the preparation of testing samples to raise the probability of the detection of disorders. Especially concerning disorders located in body regions, that are merely inaccessible, or accessible only under circumstances, that are quite consuming or discomfortabel to patients alternative methods for reliable detection of molecular markers associated with disorders are desirable.

The method as is claimed according to the present invention fulfils the needs present in the prior art as presented above.

According to the present invention immunological entities produced by the immune system of individuals directed against novel peptides, that arose from frameshift mutations in coding microsatellite regions, may be used for the detection of the presence of disorders within the body of individuals.

The invention is based on the inventors findings, that new peptide sequences, arising from frameshift mutation in coding microsatellite regions, may induce immune response in organisms, and that furthermore the components of the immune system specifically directed against said peptides may be detected within body fluids.

It is known in the art, that the mutant proteins or peptides derived from aberrantly expressed proteins may elicit a specific cellular immune response and thus may be recognized by cytotoxic T lymphocytes (CTL). This is especially true concerning mutations resulting from chromosomal instabilities, but pertains also to the more subtle genetic alterations, such as small deletions and insertions in microsatellites.

The T-cell mediated immune response on the other hand provides a strong and specific selection pressure for the rapidly growing tumour cells (Tomlinson I, et al., Nat. Med. 1999; **5**: 11-2). Thus tumour evolution is forced to circumvent the immune surveillance by the cellular immune response. As a consequence of this selection pressure, mutations in genes of the antigen processing or presenting machinery arise in DNA mismatch repair (MMR)-deficient tumour cells. In fact evasion of the immune surveillance by acquiring β2-microglobulin mutations has been observed at high frequency in MSI⁺ tumour cells (Bicknell DC, et al., Curr. Biol 1996; **6**: 1695-7). Other targets of specific mutation or down-regulation of expression are TAP1/TAP2 or HLA alleles. Direct down-regulation of expression of immunogenic epitopes is also a possible mechanism of immune escape as has been shown for melanoma-associated antigens in vivo (Jager E, et al., Int. J. Cancer 1997; **71**: 142-7). Due to this fact the relevant epitopes may not be detectable by the immune system. Thus it was suspected, that components of the immune system directed against novel peptides, characteristic for e.g. tumor cells, are not suitable for the detection of the disorders especially in the case of frameshift mutation peptides. This is due to the fact, that MSI disorders are frequently associated with DNA mismatch repair deficiency and thus are especially prone to mutations. This makes the affected cells especially apt to escape mutations in response to the attack by the immune system.

The inventors have now surprisingly found, that specific antibodies or antigen recognizing cells directed against a particular new frameshift peptide are in detectable levels present within the body fluids of individuals harbouring MSI associated disorders. This is especially due to the fact, that during specific stages of tumour development cells express the aberrant proteins. During these stages the peptides may be accessible to the immune surveillance, and thus an immune response may be elicited. Even though cell populations affected by respective disorders such as tumours may be eliminated from the organism, or may be mutated, such, that no further presentation of immunogenic epitopes may occur, there remains an immunological memory of the presence of the respective peptides at a certain time. Such the evolution of a cell population affected by a disorder associated with the expression of new frameshift peptides leaves immunological traces of its existence consisting e.g. of antibodies and specific T-lymphocytes directed against the particular peptides. Hence the presence of specific immune response elements directed against a particular frameshift peptide is indicative of the presence of a population of (tumour) cells, that expresses or has expressed at a certain time the respective peptide.

To further enhance the fidelity of the detection of the presence or absence of a disorder a set of peptides frequently occurring in disorders such as tumours may be applied in the detection reaction.

The present invention thus provides a method for the detection of disorders associated with frameshift peptides arising from mutations in coding microsatellite regions based on the detection of specific immunological entities directed against said frameshift peptides present in the body fluids of affected individuals directed against said frameshift peptides. The method is suited for primary detection of disorders such as tumours, for the early detection of disorders or of precursory stages of disorders, and for the assessment of prognosis in said disorders.

The method according to the present invention may be used for the detection of disorders associated with frameshift mutations within coding microsatellite regions of genes. These disorders comprise for example degenerative diseases, such as neurodegenerative diseases, vascular disorders, disorders caused by stress, such as oxidative stress, chemically induced stress, irradiation induced stress, etc. and cancers including all sporadic cancers as well as HNPCC associated cancers. Cancers as used herein may comprise e.g. colorectal cancer, small cell lung cancer, liver cancer (primary and secondary), renal cancer, melanoma, cancer of the brain, head and neck cancer, gastrointestinal cancers, leukemias, lymphomas. prostate cancer, breast cancer, ovary cancer, endometrial cancer, lung cancer, bladder cancer etc..

The method according to the present invention may be applied to any eucaryotic organisms exhibiting an immunologic defense system. The eukaryotic organisms are for example mammalian animals and especially animals of agricultural value such as pigs, cows, sheep, etc., companion animals, such as cats, dogs, horses etc., animals employed in research purposes such as mice, rats, rabbits, hamsters etc. or humans.

Immunological entities as used in the context of the present invention shall comprise any components of the mammalian immune system, that are able to specifically react with an antigenic epitope. Such immunological entities may comprise for example antibodies, all immunoglobulins, such as e.g. IgG, IgM, IgA, IgE, IgD, specific CD8+ T-cells or specific T-helper cells.

Frameshift polypeptides as used herein shall comprise any polypeptides or fragments thereof, that arise by a frameshift mutation within a coding microsatellite sequence of a gene. A gene may harbour one or more coding microsatellite regions, that may give rise to frameshift peptides. The coding microsatellites according to the present invention comprise mononucleotide repeats, dinucleotide repeats, trinucleotide repeats, tetranucleotide repeats and pentanucleotide repeats of any length. The repeats according to present invention contain preferably at least 3 and more preferably at least 5 repeats of the respective nucleotide pattern (1-5 nucleotides, which are repeated). The frameshift polypeptides as used according to the present invention comprise at least 1, more preferred at least 2 and even more preferred at least 3 amino acids of the mutated part of the polypeptide. Additionally the frameshift polypeptides may comprise fragments of the originally non-mutated proteins and/or may be fused to any other polypeptide sequences suitable for the purposes of the present invention. Examples of such polypeptide sequences are linker sequences, or structural peptide sequences, such as beta barrels, loop sequences etc. that facilitate the immunogenicity of the frameshift sequences according to the present invention within the fusion polypeptide.

Frameshift polypeptides suitable for the method disclosed herein have to be immunogenic polypeptides. This requires, that the polypeptides may stimulate immune responses in host organisms either in the form the polypeptides adopt in their natural environment and/or especially in the form the polypeptides adopt after processing by the cellular antigen processing and presenting machinery.

In certain embodiments of the present invention, especially for the detection of specific antibodies directed against frameshift polypeptides, the frameshift polypeptides themselves may be employed. Immunogenic portions as used herein is a portion of a protein, that is recognized by a B-cell and/or T-cell surface antigen receptor. The immunogenic portions comprise at least 5 amino acid residues, more preferably at least 10 amino acid residues and most preferably at least 15 amino acid residues of the frameshift polypeptides according to the present invention.

Immunogenic portions useful for the detection of specific antibodies may be provided as oligopeptides or as part of larger proteins. This is dependent on the embodiment of the invention. Where antibodies are to be detected, the antigenic epitopes may either be primary structures of polypeptides or the epitopes may be built by complex arrangements of tertiary structures of polypeptides. Concerning cells directed against specific frameshift peptides the relevant immunogenic portions are merely short fragments of peptides with a length of about 10 - 20 amino acids. Thus depending on the particular detection method the immunogenic portions have to be chosen.

In one embodiment of the invention a set of frameshift polypeptides is used for the detection of antibodies. The set may be a combination of the relevant peptides in solution, in cases, where information about the presence of immunological entities in general is sought. In contrast, when information about the presence or absence of particular frameshift peptides within a set of peptides is sought, the frameshift peptides may for example be tested each as a single, simultaneously in multiple testing reactions. Such experiments may for example be carried out in form of multi-well tests or using peptide arrays etc.

The method for detection of disorders according to the present invention thus may employ the detection of immunological entities directed against one single frameshift peptide or the detection of a set of immunological entities. The use of a multiplicity of potential mutations raises the probability to detect the presence of a particular disorder, and may furthermore give additional information useful in stratification of a disorder, in monitoring the disease course or in assessment of prognosis concerning the disease course.

In order to address a representative choice of mutations potentially characterizing a disorder a set of frameshift polypeptides used according to the method disclosed herein comprises for example 5-20, in a preferred embodiment 10-30, in another preferred embodiment 20-50, in a more preferred embodiment 50-100, in an even more preferred embodiment 100-500 and in the most preferred embodiment more than 500 different frameshift polypeptides originating from frameshift mutations in coding microsatellite regions. The frameshift polypeptides to be used as a set according to the present invention are selected with respect to a number of parameters characterizing said polypeptides.

One crucial aspect influencing the selection of the frameshift peptides is the mutation frequency of the relevant microsatellite region and thus the frequency of a particular expressed frameshift polypeptide. Mutation frequency as used in the context of the present invention pertains to the percentage of samples within a defined range of total samples, which show a particular mutation. Any method suitable for the determination of the percentage of individuals in a range of samples displaying the existence of a particular genotype or phenotype (with respect to the expression of polypeptides) may be employed for the determination of the frequency according to the present invention. The frequencies may be determined e.g. as described below in example 4.

The frequency may be the frequency of frameshift mutations within a coding microsatellite region or a frequency of expression of a frameshift polypeptide. Furthermore the frequency may be e.g. a frequency over a total of different tumour entities, the frequency with respect to a particular tumour entity, a frequency over several tumours entities restricted to particular stages of tumourigenesis or a frequency with respect to a particular tumour entity restricted to particular stages of tumourigenesis.

The frequency according to the present invention has to be determined taking into account a range of samples sufficient to render significant data. For example preferably at least 50 to 100 tumours may be included in the range of samples analysed. In case, that a smaller number of samples has been taken into account for determination of the frequency, a variation of the determined frequency may take place, if the range of samples is broadened.

Using frameshift polypeptides, that occur in a very large percentage of tumours, the probability, that a particular tumour expresses the peptide and may thus give rise to antibodies or antigen-recognizing cells, increases. By combination of multiple such polypeptides, the probability to address particular tumour cells further is raised. Preferred frameshift polypeptides according to the present invention occur in at least 25%, more preferred frameshift polypeptides in at least 30% and most preferred frameshift polypeptides in at least 33% of the cases of the particular condition to be detected by the method according to the present invention. For example a set of 10 polypeptides, each occurring with a frequency of more than 30 percent will statistically cover a range of more than 95% of the potentially existing disorders, as far as they have been included in the studies leading to the respective frequencies.

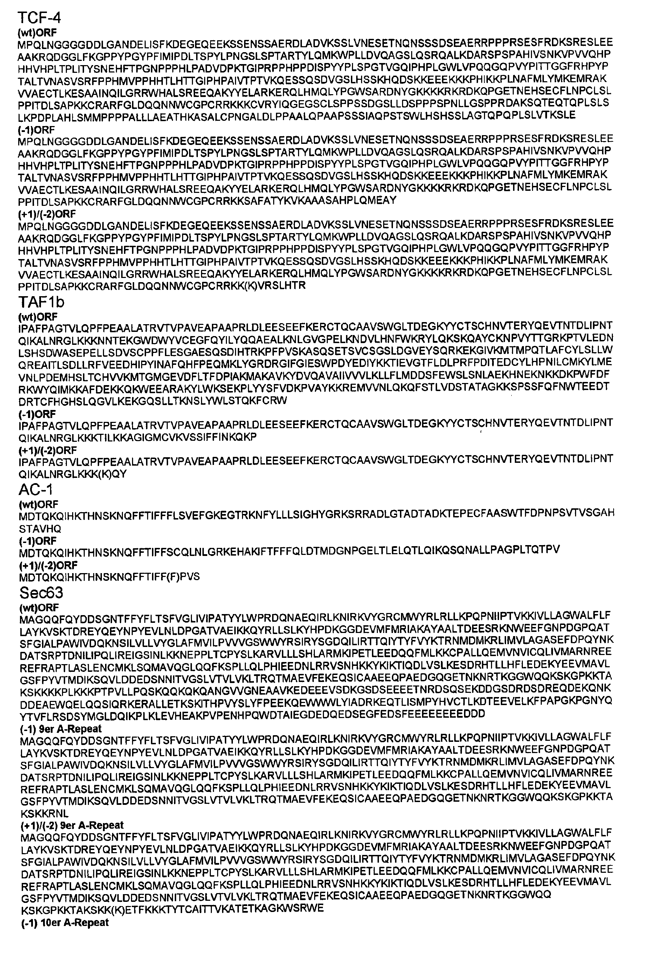

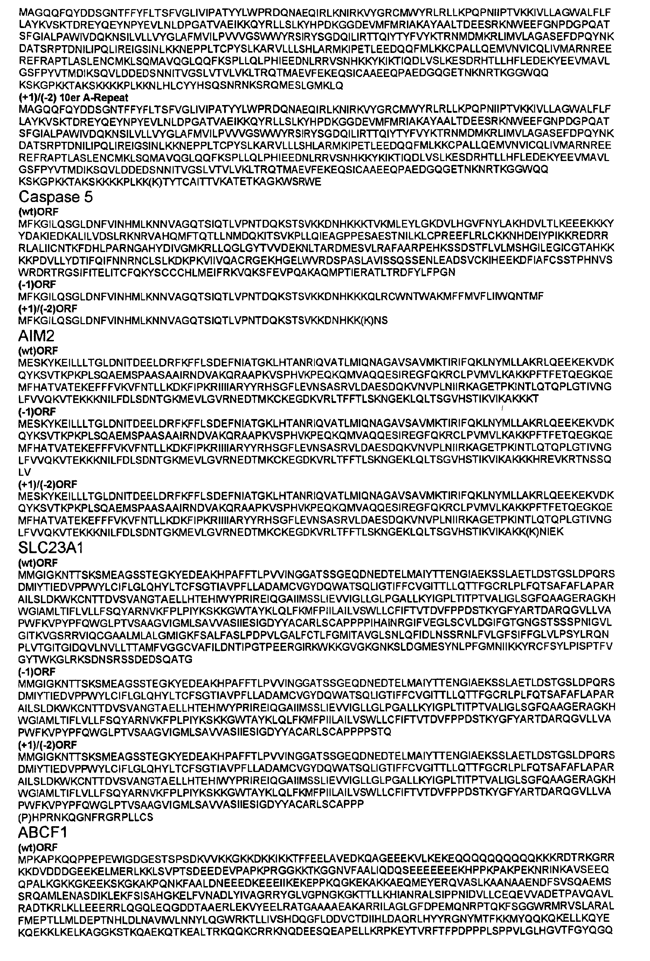

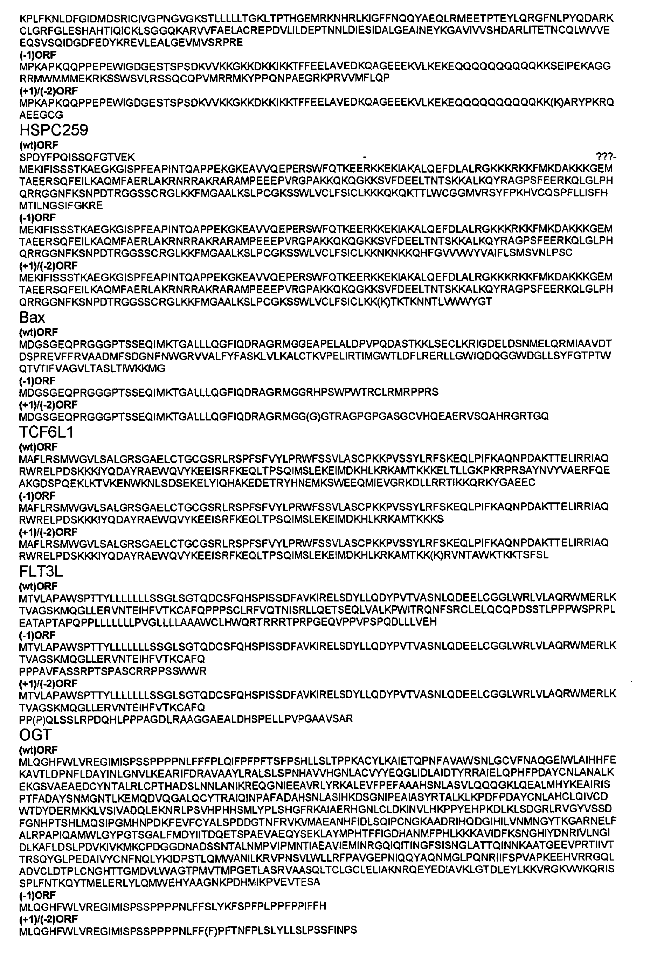

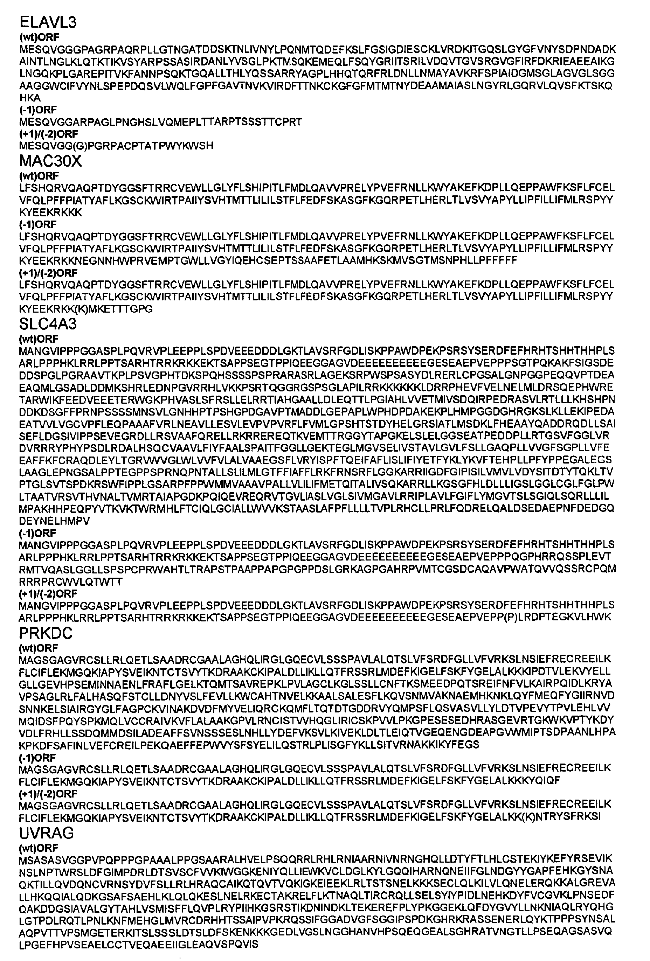

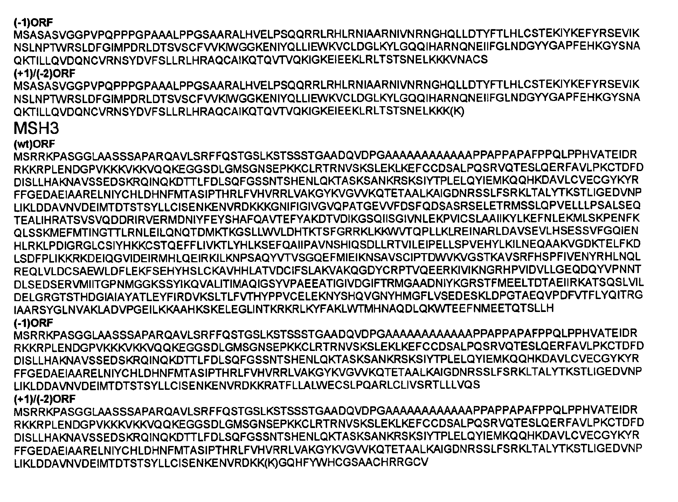

A second aspect influencing the choice of a suitable set of frameshift polypeptides concerns the type of mutation found in the microsatellite region (Polypeptides encoded by genes with coding microsatellites and the respective polypeptides encoded by genes with frameshift mutations are given in Figure 5). The most common frameshift mutation in coding microsatellites is the -1 mutation. In these mutations one nucleotide is deleted such that the reading frame is shifted by one nucleotide behind the original reading frame. This type of mutation renders a reading frame identical to that produced +2 mutations, which arise from two nucleotide insertions in the respective microsatellites. The respective polypeptides differ by one amino acid. The other mutation variant leading to frameshift mutations differing from -1 mutations concerning the resulting reading frame is the +1 mutation, arising from one nucleotide insertion, thus rendering a reading frame one nucleotide in front of the wild-type reading frame. This mutation type gives a reading frame identical to that of -2 mutations, wherein the encoded polypeptides differ by one amino acid. -3 and +3 mutations are irrelevant according to the present invention, for they do not give rise to frameshift polypeptides. According to the present invention the polypeptides included within the set shall be selected as to cover the widest possible range of disorders. Thus a set comprises for example -1 frameshift polypeptides and additionally +1 frameshift polypeptides. Using more than one possible novel reading frame of the particular gene broadens the spectrum of addressed cells and thus may prevent escape of particular cells from being detected according to the present invention.

A further aspect influencing the choice of the member polypeptides included in a set of frameshift polypeptides according to the present invention is the involvement of the gene encoded for by the coding sequence containing the respective microsatellite in particular biochemical pathways. The term biochemical pathway is used with a rather broad meaning herein. Biochemical pathways as used within this document shall for example include signal transduction pathways, enzymatic pathways, apoptosis pathways, DNA repair or polymerization pathways, the pathway of meiosis etc.. To broaden the spectrum of disorders to be addressed by the set of frameshift polypeptides, members of different pathways are included in the set. In a preferred embodiment at least 3 different pathways, in a more preferred embodiment at least 4 different pathways and in the most preferred embodiment at least 5 different pathways are represented by the frameshift polypeptides in a set according to the present invention. For example the TGFβRII as a member of a signal transduction pathway may be used in combination with the BAX gene as a member of the apoptosis pathway with additional other polypeptides associated with other pathways.

A final aspect influencing the choice of suitable frameshift polypeptides to be included in a set according to the present invention is the length of the novel (poly)peptide sequence arising from the mutation. The shift of the reading frame leads to novel stop codons. Thus the new peptides are not of the same length as the polypeptides naturally encoded by the particular gene. In most cases the new peptides are shorter or even drastically shorter than the original wild-type polypeptide. Frequently rather oligopeptides arise from the frameshift mutations. The fidelity of the immune system in recognizing "foreign" molecules reaches thus far, to identify even polypeptides, that differ from "own" polypeptides in only one single amino-acid mutation. The fragments, bound by the antigen presenting HLA molecules comprise 12 amino acid residues. To enhance the fidelity of recognition of new polypeptides more than one single amino-acid difference should be present. A polypeptide comprising 3 consecutive amino acids differing from the wild-type amino acid sequence is reliably recognized as foreign by the immune system. This may be due to the increased probability of new amino acid combinations being present in different fragments produced by the antigen presenting machinery. Thus in one embodiment of the present invention the frameshift polypeptides of a set contain at least one new amino acid, not present in the wild-type polypeptide, in a more preferred embodiment at least 2 new amino acids and in the most preferred embodiment at least 3 new amino acids.

According to the named parameters a basic set of frameshift polypeptides may be tailored, that is suitable to address a large variety of antigen recognizing immunological entities and minimizes the danger of escape of single mutated cells from being detected. Thus the probability of overseeing cells affected by a disorder in an organism can be reduced and the rate of false negative results can be minimized.

A basic set of frameshift polypeptides includes frameshift polypeptides, that do occur with a high mutation frequency in associated disorders. Additionally the polypeptides within the set are chosen to be involved in different biochemical pathways. The mutation types are chosen, that polypeptides of a minimal length of 3 amino acid residues is expressed from the mutated nucleic acid sequence. Furthermore different mutation types of one single microsatellite may be included in the set if applicable.

Additional to the parameters given above, data concerning the particular disorder in focus may be taken into account for the design of particular sets of frameshift peptides according to the present invention. Thus individual mutation frequencies, typical mutation types, relevant biochemical pathways or special immunological characteristics may contribute to the set to be used in particular cases.

In certain embodiments of the present invention frameshift polypeptides may comprise fusion or chimeric polypeptides containing sequences disclosed herein. Fusion proteins comprise the frameshift polypeptide according to the present invention together with any second and further polypeptides, such as e.g. one more frameshift polypeptide of the same sequence or of another sequence. Heterologous polypeptides may comprise enzymes, receptor molecules, antigens, antigenic or immunogenic epitopes or fragments, antibodies or fragments thereof, signalling polypeptides or signal transducing polypeptides, labelled polypeptides etc.. In one embodiment of the invention the fusion peptides may be constructed for enhanced detection or purification of the frameshift polypeptides, or of complexes of the frameshift polypeptides with the respective immunological entities according to the present invention. For the purpose of purification tags, such as e.g. his-tags, myc-tags etc. may be added to the polypeptides. For the purpose of detection antigenic portions, enzymes, chromogenic sequences etc. may be fused to the polypeptides. The fusion proteins of the present invention may (but need not) include a linker peptide between the first and second polypeptides.

A nucleic acid sequence encoding a fusion protein of the present invention is constructed using known recombinant DNA techniques to assemble separate nucleic acid sequences encoding the first and second polypeptides into an appropriate expression vector. The 3' end of a nucleic acid sequence encoding the first polypeptide is ligated, with or without a peptide linker, to the 5' end of a nucleic acid sequence encoding the second polypeptide ensuring the appropriate reading frames of the sequences to permit mRNA translation of the two nucleic acid sequences into a single fusion protein that retains the biological activity (antigenicity) of both the first and the second polypeptides.

A peptide linker sequence may be employed to separate the first and the second polypeptides by a distance sufficient to ensure, that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Pat. No. 4,935,233 and U.S. Pat. No. 4,751,180. The linker sequence may be from 1 to about 50 amino acids in length. Peptide sequences are not required when the first and second polypeptides have non-essential N-terminal amino acid regions that can be used to separate the functional domains and prevent steric interference.

Mutation as used in the context of the present invention may comprise insertions or deletions of one or several nucleotides (or nucleotide repeats) within the specified microsatellite sequences. In a preferred embodiment of the present invention the number of nucleotides to be inserted or deleted is not 3 or must not be divisible by 3, such that the mutation leads to a frameshift with respect to the reading frame of the downstream nucleic acid sequence. Thus the nucleic acid sequence downstream of the mutation point will render a polypeptide sequence different from the native sequence encoded by the respective gene. The mutation in these cases leads to a novel peptide sequence (a neo-peptide). Commonly the new peptide sequence is short due to the fact, that novel stop codons arise from the shift in the reading frame.

Diagnosis as used in the context of the present invention may comprise determining the presence or absence and/or the level of specific immunological entities directed against particular frameshift peptides in a sample, and assessing diagnosis from said presence or absence and/or level of immunological entities specifically directed against said frameshift polypeptides.

Based upon the determined presence or absence and/or the levels of immunological entities specifically directed against particular frameshift peptides in the samples individuals can be subdivided into subgroups. The subgroups may be created according to scientific or clinical data, such as e.g. survival, recurrence of disease, frequency of metastases etc., related to the presence or absence and/or levels of particular frameshift peptides in samples of tissues affected with a particular disorder, of tissues being in question of being affected with a particular disorder or of tissues at risk of being affected with a particular disorder.

Based upon these subgroups an assessment of prognosis may be done. According to the subgroups the therapy of the individuals affected by the disorders (e.g. tumors) may be tailored.

Monitoring may comprise detecting the presence or absence or level of immunologic entities specifically directed against frameshift polypeptides in samples taken at different points in time and determining the changes in said levels or presences or absences. According to said changes the course of the disease can be followed. E.g. the occurrence of immunologic entities directed against frameshift peptides, that have not been present at an earlier time-point may be indicative of the progression of evolution of the affected tissue. The course of the disease may be used to select therapy strategies for the particular individual.

Another aspect of diagnosis and monitoring of the disease course according to the present invention may comprise the detection of minimal residual disease. This may comprise for example the detection of presence and/or level of immunologic entities specifically directed against said frameshift polypeptides, that have not been present in earlier examinations in one or more body samples following initial therapy of an individual once or at several timepoints. According to the presence and/or level of immunologic entities specifically directed against new frameshift polypeptides detected in the samples one may select a suitable therapy for the particular individual.

Furthermore the diagnostic method may be carried out to detect disseminated tumor cells in biological samples as diagnosis of minimal residual disease (MRD). For this purpose the detection of the level of immunological entities or the presence of immunological entities, specific for particular frameshift peptides, that have not been detected in prior examinations, may be performed.

A sample according to the method of the present invention may comprise any sample comprising immunological entities as defined above. Samples may comprise samples of clinical relevance, such as e.g. secretions, smears, body fluids, urine, semen, stool, bile, biopsies, cell- and tissue-samples. Biopsies as used in the context of the present invention may comprise e.g. resection samples of tumors, tissue samples prepared by endoscopic means or needle biopsies of organs.

Such samples may comprise for example intact cells, lysed cells or any liquids containing antibodies, immunoglobulins or cells specifically directed against frameshift peptides. Even solids, to which cells, cell fragments or antigen binding polypeptides, such as antibodies or immunoglobulins may adhere, or may be fixed, may be samples according to the method disclosed herein. The method for detection of the level of the immunological entities according to the present invention is any method, which is suited to detect very small amounts of specific immunological entities in biological samples. The detection reaction according to the present invention is a detection either on the level of antibodies or on the level of cells specific for particular antigens.

The detection may be carried out in solution or using reagents fixed to a solid phase. Solid phases may comprise beads of a variety of materials, such as e.g. agarose, dextrane polymers, polystyrene, silica, etc. or surfaces of suitable materials such as e.g. polystyrene, glass, agarose, protein, dextran etc. coated surfaces etc.. The detection of one or more immunological entities, such as immunoglobulins or cells carrying specific antigen recognizing epitopes, with different antigen binding specificities may be performed in a single reaction mixture or in two or more separate reaction mixtures. Alternatively the detection reactions for several immunological entities may for example be performed simultaneously in multi-well reaction vessels.

The immunological entities specifically recognizing particular frameshift peptides may be detected using reagents that specifically recognise these immunological entities alone or in complex with their respective antigen (e.g. antibodies), or reagents, that are specifically recognized by the immunological entities themselves (e.g. the antigen). In one embodiment the antigen may be fused to another polypeptide, so as to allow binding of the antigen by the immunological entity in question and simultaneously binding of the second part of the fusion protein by another (labelled) antibody for the detection. The detection reaction for the immunological entities may comprise one or more reactions with detecting agents either recognizing the initial entities or recognizing the prior molecules used to recognize the immunological entities.

The detection reaction further may comprise a reporter reaction indicating the presence or absence and/or the level of the immunological entities. The reporter reaction may be for example a reaction producing a coloured compound, a bioluminescence reaction, a chemiluminescent reaction, a fluorescence reaction, generally a radiation emitting reaction etc.. The detection reactions may for example employ antibodies or binding reagents that are detectably labelled.

Furthermore the binding of detection molecules to the entities in question may be detected by any measurable changes in physical or physical-chemical properties, such as changes in spectroscopic properties, in magnetic resonance properties etc.. Different immunological entities or immunological entities of different specificities may be recognized by different methods or agents. This may be due to difficulties in detection of several entities, or entities with particular specificities by a particular method. An advantage of the use of different detection techniques for different immunological entities or for immunological entities with different specificities may for example be, that the different reporter signals referring to different immunological entities could be distinguished.

Generally in a method according to the present invention the detection of different immunological entities such as the detection of immunoglobulins and the detection of immunocompetent cells may be performed simultaneously.

For all detection purposes optionally the original sample may be concentrated by any suitable means known to those of ordinary skill in the art. Furthermore steps may be involved to selectively extract immunological entities from the sample mixture such as affinity based purification techniques either employing specific antibodies or the respective antigen recognized by the entities in question.

Applicable formats for the detection reaction according to the present invention may be, (reverse) blotting techniques, such as Western-Blot. The blotting techniques are known to those of ordinary skill in the art and may be performed for example as electro-blots, semidry-blots, vacuum-blots or dot-blots. Furthermore immunological methods for detection of molecules may be applied, such as for example immunoprecipitation or immunological assays, such as ELISA, RIA, Elispot assay, lateral flow assays, immuno-cytochemical methods etc..

In one preferred embodiment of the invention the detection of the level of immunological entities specific for frameshift peptides is carried out on the level of antibodies. This may be e.g. performed using the specific interaction between the respective frameshift peptides with the antibodies. The determination of the presence or absence and/or the level of the antibodies may for example be carried out with recombinantly produces frameshift peptides. The peptides can be used in many different detection techniques for example in western-blot, ELISA or immunoprecipitation. In one embodiment the detection of antibodies is carryout as antibody capture assay (Antibodies A laboratory Manual, Harlow, Ed. et al., Cold Spring Harbor Laboratory 1988).

In another embodiment of the invention the detection of the specific antibodies is carried out using monoclonal or polyclonal antibodies specifically recognizing the antigen binding epitope of the first antibodies. For this purpose the above mentioned immunological detection procedures may be applied. In a further embodiment chimeric antigens may be employed in the detection reaction. Such chimeric antigens may for example comprise fusion proteins combining the antigenic epitope of a frameshift polypeptide, recognized by the antibody in question, fused to another antigen, that may be recognized by a detection antibody. The particular antigens within the chimeric polypeptide may be separated by a linker or spacer region.

Any other method for determining the amount of particular antibodies or immunoglobulins in biological samples can be used according to the present invention.

Generally the detection of the antibodies according to the present invention may be performed as well in vitro as directly in situ for example in the course of an immuno-histochemical or immuno-cytochemical staining reaction.

Cells exhibiting specificity for a particular antigen may be detected by any methods suitable for that purpose known to those of ordinary skill in the art. Methods may for example comprise·proliferation-assays, cytokine-ELISAs, ELISpot assays, intracellular FACS-staining, PCR-mediated identification of peptide-specific cytokine (or similar) - expressing cells, tetramer-staining, cytotoxicity assays and DTH-(delayed type hypersensitivity) reactions.

In case of proliferation-assays induction of peptide-specific T-cell proliferation may be measured by methods known to those of skill in the art. This can be achieved by simply counting of cells, by measuring incorporation of labelled nucleotides into cellular DNA or by measuring level and/or activity of cellular protein(s). Cytokine-ELISA may comprise identification of peptide-specific cytokine-secreting cells by measuring cytokine levels in supernatant. In the course of an ELISpot assay the number of peptide-specific cytokine (i.e. IFN-γ)- secreting cells in a sample is determined. Similarly the Intracellular FACS-staining identifies cytokine-expressing cells on the protein level. In contrast (real-time) PCR may be used for identification of peptide-specific cytokine (or similar) -expressing cells on the transcript level. In the course of a tetramer-staining assay the label is a tetramer-molecule of recombinant MHC-class I molecules, loaded with specific peptide and coupled to a dye. The tetramer binds to the T-cell receptor. Cytotoxicity assays are a method for identification of cells, that can recognize and kill target cells in a peptide-specific manner. DTH-(delayed type hypersensitivity) reaction is based on the measuring of skin-reaction of vaccinated persons after intradermal (or similar) application of peptide(s).

Another aspect of the present invention is a testing kit for performing the method according to the present invention. The kit may be for example a diagnostic kit or a research kit.

A kit according to the present invention comprises at least an agent suitable for detecting the immunological entities according to the method disclosed herein. Furthermore a kit according to present invention may comprise:
a) reagents for the detection of the antibodies or cells specifically recognizing antigens.
b) reagents and buffers commonly used for carrying out the detection reactions as described herein, such as buffers, detection-markers, carrier substances and others
c) a sample for carrying out a positive control reaction, that may comprise an antigen or a set of antigens, to which almost all members of a target population of individuals have antibodies.

The reagent for the detection of the antibodies or cells specifically recognizing antigens may include any agent capable of binding to the antibodies or cells specifically recognizing antigens. Such reagents may include proteins, polypeptides, nucleic acids, peptide nucleic acids, glycoproteins, proteoglycans, polysaccharids or lipids.

The sample for carrying out a positive control may comprise for example an antigenic peptide or a set of antigenic peptides. Suitable antigens may include antigens, against which a wide percentage of the population has antibodies. Examples of such antigens may for example comprise antigens present in lysed E. coli cells, tetanus antigen, the viral capsid antigen of the Epstein-Barr virus, antigens derived from matrix proteins of Haemophilus influenzae. The antigens may for example be used as a mixture to ensure, that a particular individual actually displays a positive reaction.

In a preferred embodiment of the invention the detection of the immunological entities directed against particular frameshift polypeptides is carried out on the level of antibodies. In this embodiment the binding agent may be for example a frameshift polypeptide or a fragment thereof, recognized by the respective antibody, or a fusion polypeptide comprising said frameshift polypeptide or a fragment thereof. Furthermore the binding agent may comprise an antibody or a fragment thereof specific for the antibody in question, for the complex of the antibody with the respective frameshift polypeptide or for an antigenic epitope fused to the frameshift polypeptide.

In another embodiment of the test kit the detection of the immunological entities is carried out on the level of cells specifically recognizing frameshift polypeptides. In this embodiment of the invention the reagent for the detection may be for example a frameshift polypeptide or a fragment thereof, recognized by the respective antibody or T-cell receptor, or a fusion polypeptide comprising said frameshift polypeptdie or a fragment thereof. Furthermore the binding agent may comprise an antibody or a fragment thereof specific for the antibody in question, for the complex of the antibody with the respective frameshift polypeptide or for an antigenic epitope fused to the frameshift polypeptide.

### Brief description of the drawings:

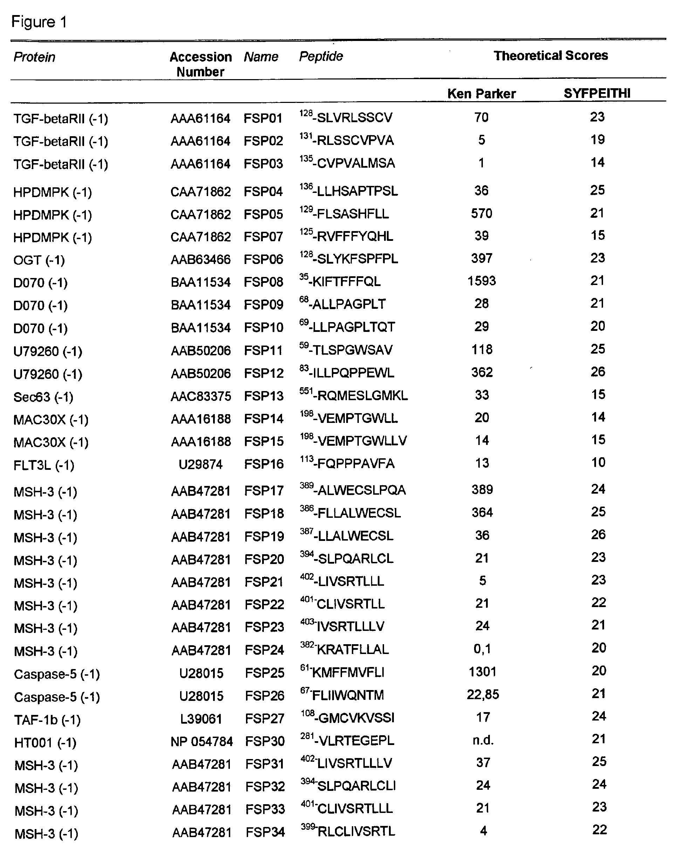

- Figure 1:: **Frameshift peptides analysed for in vitro stimulation of a cellular immune response;** All analysed peptides are derived from (-1) mutations in microsatellites of the cognate proteins; The protein or nucleotide accession numbers are indicated within the table; the position of the start amino acid in the protein is indicated in the tables; the predicted binding scores to HLA-A2.1 using computer assisted analysis; for experimental details see Example 1

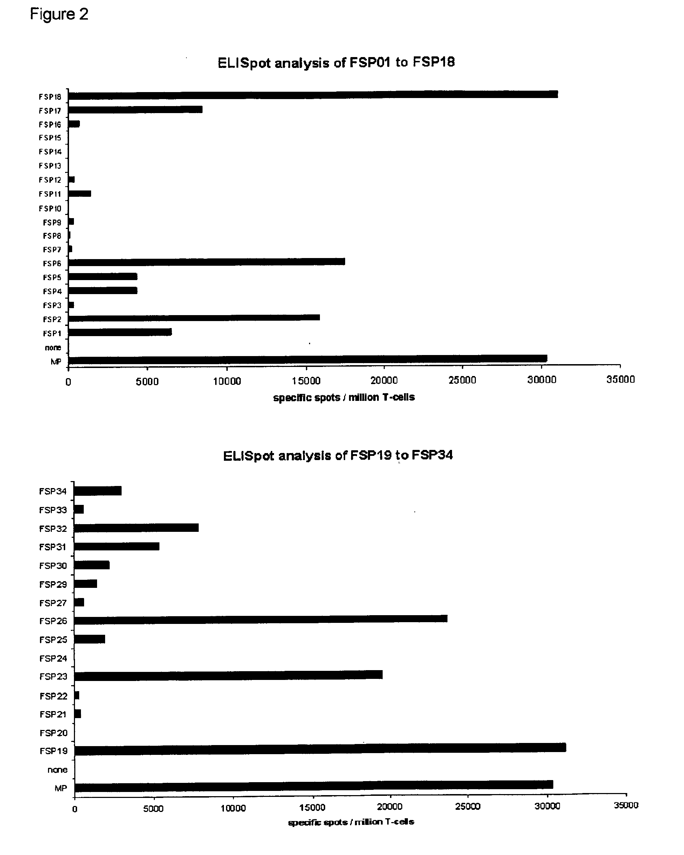

- Figure 2:: **ELISpot-analysis of FSP T-cell lines** (FSP01-FSP34). Titrated amounts of T-cells were incubated overnight with 3.5x10⁴ peptide loaded T2 cells per well. The number of IFN-γ-releasing activated T -cells (spots) among the total number of cells analyzed (10⁶) is depicted for each frameshift peptide. For experimental details see Example 1.

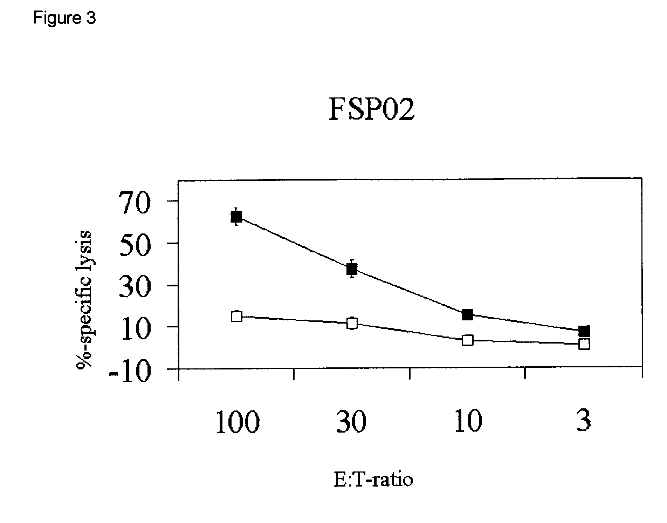

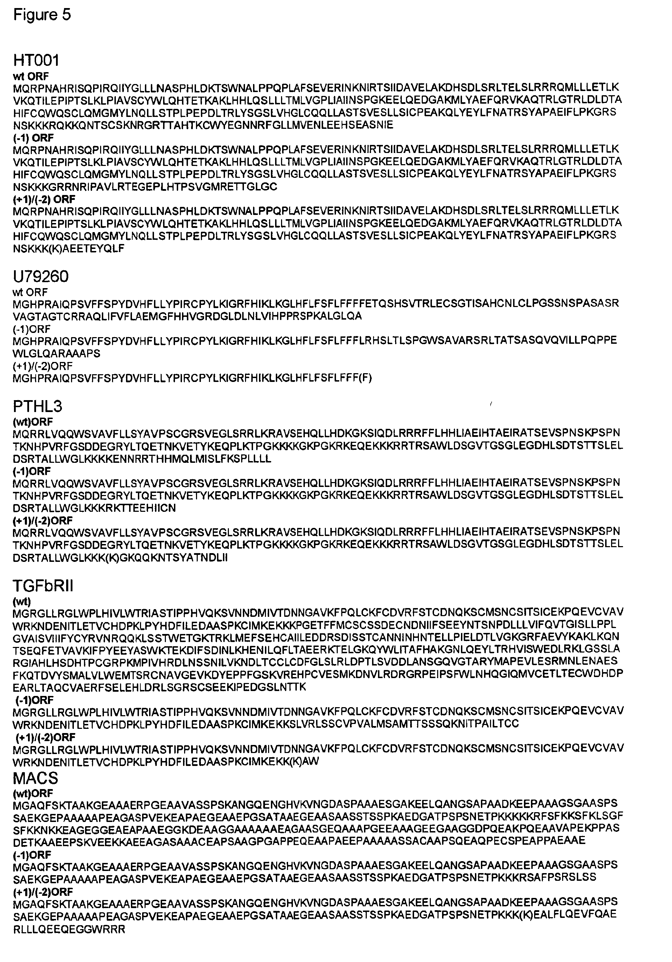

- Figure 3:: **Frameshift peptide specific and HLA-A2-restricted lysis of target cells. (FSP02);** The antigen specificity of the FSP02 CTL line was tested in the presence of unlabeled cold targets, T2 cells pulsed with FSP02 (open squares) at an inhibitor:target ratio of 50:1. Lysis without cold targets is shown as a control (closed squares). All data are shown as the mean and standard deviation from 3 replicate wells. For experimental details see Example 2.

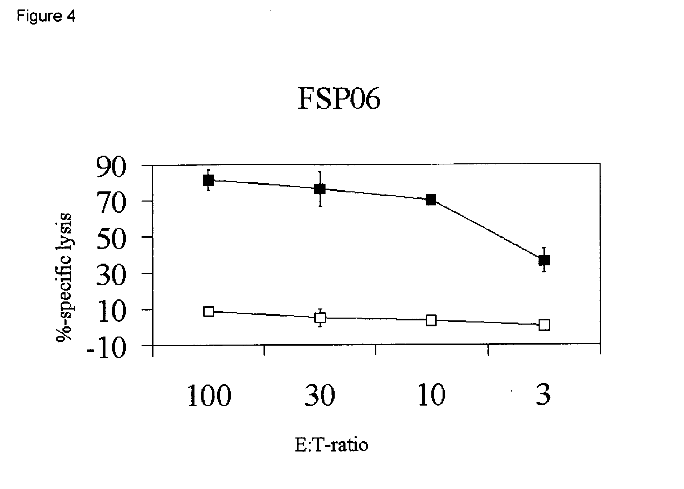

- Figure 4:: **Frameshift peptide specific and HLA-A2-restricted lysis of target cells. (FSP06);** The antigen specificity of the FSP06 CTL line was tested in the presence of unlabeled cold targets, T2 cells pulsed either with FSP06 (open squares) at an inhibitor:target ratio of 50:1. Lysis without cold targets is shown as a control (closed squares). All data are shown as the mean and standard deviation from 3 replicate wells. For experimental details see Example 2.
- Figure 5:: **Listing of sequences of polypeptides encoded by genes with coding microsatellites;** the sequences of polypeptides arising from different possible frameshift mutations are depicted. for each polypeptide the sequence expressed from the wild type open reading frame is given (wtORF); Furthermore the sequences expressed from (-1) mutations and, from (-2/+1) mutations are given.

### Example 1: In vitro stimulation of cellular immune response by frameshift peptides

The present experiments were performed in order to determine, whether the frameshift peptides arising from mutations in coding microsatellite regions according to the present invention are suited to stimulate a cellular immune response. The experiments were performed as follows:

Peptides displaying HLA-A2.1-binding motifs were selected by taking advantage of specific computer programs [(Parker, Bednarek, & Coligan 1994); http://bimas.dcrt.nih.gov/molbio/hla_bind/ and (Rammensee et al. 1999); http://134.2.96.221/scripts/MHCServer.dll/home.htm]. Peptides were purchased from the peptide synthesis unit of the DKFZ. Stock solutions (10mg/ml in DMSO) were stored at -70°C and diluted to 1mg/ml in PBS before use. T2 cells were pulsed with 50µg/ml peptide and 5µg/ml β2-microglobulin (Sigma; Deisenhofen, Germany) overnight at 37°C. The expression of HLA-A2.1 was then analysed by flow cytometry using mAb BB7.2 followed by incubation with FITC-conjugated (Fab')2 goat antimouse Ig (Vonderheide et al. 1999).

Peripheral blood was obtained from a healthy HLA-A2.1+ donor and collected in heparinized tubes. PBMNC were isolated by Ficoll-density gradient centrifugation. Whole CD3+ T-cells were isolated from PBMNC by magnetic depletion of non-T-cells using the MACS Pan T-Cell Isolation Kit (Miltenyi; Bergisch Gladbach, Germany) according to manufacturer's instructions. Preparations contained at least 97% of CD3+ cells as assessed by immunophenotypic analysis.

CD40 Bs of a HLA-A2.1+ donor were incubated with peptide (10µg/ml) and human β2-microglobulin (3µg/ml; Sigma) in serum-free Iscov's DMEM medium for one hour at room temperature, washed twice to remove excess of peptide, were irradiated (30Gy) and added to purified CD3+ autologous T-cells (>97% CD3+) at a ratio of 4:1 (T:CD40 Bs) in Iscov's MEM containing 10% human AB-serum, supplements (1:100) and hlL-7 (10ng/ml, R&D). Cells were plated at a density of 2 x 10⁶ T-cells/well in 1 ml of medium. After three days in culture they were fed with 1ml complete medium. For restimulation of T-cells, this was repeated weekly. IL-2 was first given at day 21 (10IU/ml, R&D), also at day 24 and from day 28 on only hlL-2 was used instead of hlL7.

ELISpot assays were performed as described elsewhere (Meyer et al. 1998). Briefly, nitrocellulose-96-well plates (Multiscreen; Millipore, Bedford, USA) were covered with mouse anti-human IFN-y monoclonal antibody (Mabtech, Sweden) and blocked with serum containing medium. Varying numbers of effector cells were plated in triplicates with 3,5x10⁴ peptide-loaded T2 cells per well as targets. After incubation for 18h, plates were washed, incubated with biotinylated rabbit anti-human IFN-γ second antibody, washed again, incubated with streptavidin coupled alkaline phosphatase, followed by a final wash. Spots were detected by incubation with NBT/BCIP (Sigma) for 45min, reaction was stopped with water, after drying spots were counted using the KS-ELISpot reader (Zeiss Kontron; Göttingen, Germany).

These procedures were performed for peptides derived from mutations in the coding regions of the following genes: TGFβRII, OGT, U79260, CASP 5, MSH 3, HPDMPK, HT001, TAF1B, D070, MAC30X, FLT3L and SEC63. Peptides are listed in Figure 1. Results for ELIspot analysis are shown in Figure 2.

The analysis shows, that the used peptides are suited to raise an immune response. Peptides arising from frameshift mutations thus may be used to raise immune response for example in the course of vaccinations according to the present invention.

### Example 2: Cytotoxicity assay directed against cells displaying frameshift peptides.

CTL bulk cultures and/or CTL clones obtained according to the method described in Example 1 were tested for their cytotoxicity as follows:

Due to the limited amount of cell material clones were in some experiments pooled for the determination of the cytotoxicity.

To obtain cells presenting frameshift peptides on the one hand different MSI+ cell lines endogenously expressing mutated mRNA of the respective frameshift peptides of TGFβRII, OGT, U79260, CASP 5, MSH 3, HPDMPK, HT001, TAF1B, D070, MAC30X, FLT3L and SEC63 either expressed HLA-A2.1 endogenously or were stably transfected with HLA-A2.1, on the other hand several MSI- cell lines expressing HLA-A2.1 were transfected with the mutated full-length cDNA of the respective frameshift peptides (of TGFβRII, OGT, U79260, CASP 5, MSH 3, HPDMPK, HT001, TAF1B, D070, MAC30X, FLT3L and SEC63). After selection and expansion of the transfected cell lines for each respective frameshift peptide at least two stably transfected sub-cell lines were available. These cell lines were used in cytotoxicity assays, wherein negative controls were the respective untransfected MSI+ HLA-A2.1 negative and/or the MSI-, HLA-A2.1 positive cell lines.

It could be shown, that the transfected cell lines were lysed by the bulk cultures and/or pooled clones of CTLs. The reactivity was tested at different target cell to effector cell ratios. In average around 20%-30% of the target cells were lysed in the assays. The control cells were always lysed at a significantly lower percentage.

In Figures 3 and 4 results for the frameshift peptides FSP02 (TGFβRII(-1)) and FSP06 (OGT(-1)) respectively are shown. These results shall be representative for the results related to the other frameshift peptides, which rendered similar rates of lysis.

The experiments show, that frameshift peptides may generate immune response. The frameshift peptides may furthermore be applied for the detection of the presence of cytotoxic T-cells directed against a particular frameshift peptide.

### Example 3: Screening for Antibodies directed against frameshift peptides in patient samples

Serum of 25 patients with diagnosed colorectal carcinomas was tested for the presence of antibodies to a set of frameshift peptides arising from frameshift mutations in coding microsatellite regions of the following genes: TGFβRII, U79260, CASP 5, HT001, PTHL3, MACS, TCF4, TAF1B, AC1, AIM2, SLC23A1, ABCF1, HSPC259, BAX, TCF6L1, FLT3L, OGT, ELAVL3, MAC30X, MAC30X, SLC4A3, PRKDC, UVRAG, MSH3 and SEC63. As a control the serum of 50 normal individuals was tested.

For the test synthetic peptides (20 to 40mers, partly overlapping) representing immunogenic portions of all relevant frameshift peptides (see figure 5) arising from the respective genes were spotted onto nylon membranes. The nylon membranes were subsequently incubated for one hour in phosphate-buffered saline (PBS) with 5% milk powder for blocking unspecific membrane binding. After washing the membranes 3x with PBS, the membranes were incubated with the test and control sera. The sera were diluted 1:1.000 in PBS/0,5% milk powder and incubated overnight with gentle shaking. Subsequently the sera were removed, and membranes were washed three times in PBS before they were incubated with a polyclonal alkaline phosphatase conjugated goat anit-human IgG antibody for one hour. Thereafter, the membranes were washed repeatedly with PBS/0,05% TWEEN20 before staining reaction was developed using nitroblue tetrazolium chloride and bromochoro-indoyl-phosphate (SigmaAldrich) in Tris-buffered saline (TBS). Binding of human antibodies specific for individual frameshift polypeptides thus was made visible by color-deposit on the respective membrane.

The results show, that in all samples of tumour patients antibodies directed against at least one peptide arising from frameshift mutations were present.

Strong signals for the detection of particular antibodies could be found using frameshift peptides derived from the (A)₁₁ tract of the MACS gene, the (A)₁₀ tract of the CASP5 gene, the (A)₉ tract of the TCF-4 gene, the (G)₈ tract of the IGFIIR gene, the (T)₁₀ tract of the AC1 gene, the (A)₉ or (A)₁₀ tract of the SEC63 gene, the (A)₁₁ tract of the TAF1 B gene, the (A)₁₁ tract of the PTHL3 gene, the (T)₁₄ tract of the U79260 gene, the (A)₁₀ tract of the AIM2 gene, the (A)₁₀ tract of the ABCF1 gene.

This illustrates, that the method according to the present invention may be used for diagnosis of diseases associated with frameshift mutations in coding regions of genes. The above mentioned genes seem to be of special use for this diagnostic method.

### Example 4: Analysis of the mutation frequency of genes harbouring repeat tracts including data from publications and own data

In order to extract cMNRs and cDNRs, we screened the 109289 entries with human sequences in the EMBL database (EMBL Rel. 62, Mar. 2000). Using various Perl scripts (Wall, Christiansen, & Schwartz 1996), we obtained annotated coding sequence (a total of 46520 entries) and applied a rigorous redundancy check at the 98% level, thus detecting a total of 33595 coding sequences in these entries. cDNA entries were compared to genomic DNA to identify corresponding mRNA. If both information were available for a particular gene, priority was given to the genomic sequence, since repeats in cDNA might span several exons. We finally considered 22577 cDNAs (mRNAs) and 11018 coding sequences in genomic entries for further analysis.

In the coding regions, a minimum repeat length of 6 mononucleotides and 4 dinucleotides was required as lower limit the repeats. In regards to the dinucleotide repeats, all 12 different types of dinucleotide repeats were taken into consideration. cDNA and genomic DNA entries were analyzed separately. Using several filters, repeat tracts within pseudogenes, vector sequences as well as homopolymeric nucleotide stretches at the most 5' or 3' ends of sequences were excluded. All candidate sequences were stored in a relational database for further analysis. Subsequently, two independent BLASTN analyses were performed: initial database analysis was done using the BLASTN included in the HUSAR program package of the German Cancer Research Center in Heidelberg, followed by an Advanced BLAST analysis of the high throughput genomic sequencing (htgs) database (http://www.ncbi.nlm.nih.gov/). Exon-intron boundaries were determined by MALIGN analysis, matching a candidate cDNA with homologous genomic DNA sequences. Finally, true coding microsatellites were verified by direct sequencing of repeat candidates amplified from genomic DNA.

Published mutation data of genes harboring repeat tracts were collected. Numbers of tumour samples analyzed and the number of mutated samples found were sorted by gene and repeat type in a spreadsheet. Individual sample numbers and numbers of mutated samples from each single publication for each repeat tract within a gene were summarized. Subsequently tissue specific cumulative mutation frequencies were calculated. If mutation data were not reported separately for specific repeats, i.e. represented mixed data or if mutation data on tumour cell lines and primary tumour samples were shown as one item, these data were omitted. In order to minimize sampling errors only repeat mutation data obtained from at least 10 tissue samples were considered for statistical analysis.

Additionally investigations were performed regarding the mutation frequencies of unpublished coding microsatellite regions. Nine novel coding microsatellites residing in genes not yet analyzed for frameshift mutations in MSI colorectal, endometrial or gastric tumours have been examined in the course of the studies leading to the present invention. They include three genes containing A11 repeats (TAF1B, MACS, HT001), five genes with A10 repeats (CHD2, UVRAG, TCF6L1, ABCF1, AIM2) and one gene harboring a G9 repeat (ELAVL3). The MSI status of these specimen was determined using the NCI ICG-HNPCC microsatellite reference marker panel (8). PCR reactions were performed as follows:

Genomic DNA was isolated from 5-8 haematoxylin and eosin stained 5 µm sections after microdissection, using the Qiamp Tissue Kit (Qiagen, Hilden, Germany). Preparation of DNA from cell lines was performed according to standard protocols. PCR primers were designed to closely flank the target sequence, yielding short amplimeres of about 100 base pairs thus allowing precise fragment sizing and robust amplification from archival tissues (Table 1). PCR reactions were performed in a total volume of 25 µl containing 50 ng genomic DNA, 2.5 µl 10 x reaction buffer (Life Technologies, Karlsruhe, Germany), 1.5 mM MgCl₂, 200 µM dNTPs, 0.3 µM of each primer, and 0.5 U Taq DNA polymerase (Life Technologies) and using the following conditions: initial denaturation at 94 °C for 4 min, followed by 35 cycles of denaturation at 94 °C for 30 s, annealing at 58 °C for 45 s, and primer extension at 72 °C for 30 s. The final extension step was carried out at 72 °C for 6 min. PCR fragments were analyzed on an ALF DNA sequencing device (Amersham Pharmacia Biotech, Freiburg, Germany) using 6.6% polyacrylamide/ 7 M urea gels. Size, height and profile of microsatellite peaks were analyzed using the AlleleLinks software (Amersham Pharmacia Biotech). Coding microsatellite instability was scored, if smaller or larger-sized amplimeres were detected in tumour DNA compared to DNA from non-neoplastic cells. Allele intensities were determined and ratios of wild-type and novel alleles in normal and tumour tissues were calculated, defining a two-fold difference as threshold for allelic shifts. Similarly, unstable alleles in tumour cell lines were identified by comparison to 36 unmatched normal mucosae. In order to determine the predicted repeat type and length amplified coding microsatellites were subjected to Big Dye terminator cycle sequencing (Perkin Elmer, Darmstadt, Germany) and subsequent analysis on an ABI 310 sequencing device.

The frequencies of mutations in a particular microsatellite region examined herein are given in figure 1. The mutation rates are calculated with respect to the total number of samples included in the studies. The mutations frequencies resulting from studies covering a larger number of samples may be handled to be more significant then those referring to a smaller number of analysed samples.

Overall the results show, that a number of the analysed microsatellite sites is mutated with high frequency over the range of examined samples as well as over the range of published data. This suggests, that according to the present invention a set of frameshift peptides, arising from frequent mutations should be expressed in high percentage of tumours.

## Claims

1. A method for the detection of a disorder associated with frameshift mutations in coding microsatellite regions in an individual comprising
a. detection of the presence or absence and/or of the level of immunological entities specifically directed against one or more frameshift polypeptides originating from frameshift mutations in coding microsatellite regions in a biological sample, and
b. assessing diagnosis of the presence or absence of said disorder and/or prognosis of the disease course of said disorder from the presence or absence and/or the level of said immunological entities.

2. The method of claim 1, wherein the immunological entity is an antibody or a fragment thereof.

3. The method of claim 1, wherein the immunological entity is a cell specifically directed against a frameshift polypeptide.

4. The method of any one of the claims 1-3, wherein the disorder is a neurodegenerative disorder or cancer.

5. The method of claim 4, wherein the cancer is gastrointestinal cancer.

6. The method of any one of the claims 1-5, wherein immunological entities are specifically directed against at least one frameshift polypeptide derived from a frameshift mutation in the (A)₁₀ tract of the TGFβRII gene.

7. The method of any one of the claims 1-6, wherein immunological entities are specifically directed against at least one frameshift polypeptide derived from a frameshift mutation in the (G)₈ tract of the BAX gene.

8. The method of any one of the claims 1-7, wherein immunological entities are specifically directed against at least one frameshift polypeptide selected from a group consisting of frameshift peptides derived from the (A)₁₁ tract of the MACS gene, the (A)₁₀ tract of the CASP5 gene, the (A)₉ tract of the TCF-4 gene, the (G)₈ tract of the IGFIIR gene, the (T)₁₀ tract of the AC1 gene, the (A)₉ or (A)₁₀ tract of the SEC63 gene, the (A)₁₁ tract of the TAF1B gene, the (A)₁₁ tract of the PTHL3 gene, the (T)₁₄ tract of the U79260 gene, the (A)₁₀ tract of the AIM2 gene, the (A)₁₀ tract of the ABCF1 gene.

9. The method of any one of the claim 1-8, wherein the detection is carried out in vitro.

10. The method of any one of the claim 1-8, wherein the detection is carried out as an in situ immuno-cytochemical staining reaction.

11. A kit for performing the methods of any one of the claims 1-10, which is a diagnostic kit or a research kit.
